# EUROPEAN PATENT SPECIFICATION

(11) **EP 0 550 549 B1**
(45) Date of publication and mention of the grant of the patent: **23.04.1997**
(21) Application number: 91916967.2
(22) Date of filing: 20.09.1991
(51) Int. Cl.: C07H 1/08, B01J 20/32

(54) **ISOLATION OF POLYNUCLEOTIDES**
ISOLIERUNG VON POLYNUKLEINSÄURE
ISOLATION DE POLYNUCLEOTIDES

(30) Priority: 22.09.1990 GB 9020683
(43) Date of publication of application: 14.07.1993
(73) Proprietor: University of Strathclyde, Glasgow G1 1YQ, Scotland (GB)
(72) Inventor: CADDY, Brian, Univ. of Strathclyde, McCance Bldg.,, Glasgow G1 1XQ (GB); JING, Cheng, Univ. of Strathclyde, McCance Bldg.,, Glasgow G1 1XQ (GB)
(74) Representative: Horner, Martin Grenville
(86) International application number: GB9101612
(87) International publication number: WO9205181

(56) References cited:
- EP-A- 0 088 818
- EP-A- 0 235 526
- WO-A-91/02740
- US-A- 4 923 978

## Description

The present invention relates to a process for the isolation of polynucleotides (particularly oligonucleotides and DNA) from mixtures with proteins and materials for use in the process. In particular, the invention is concerned with the isolation of DNA from mixtures of DNA and cellular proteins produced by the lysis of lymphocytes (white blood cells).

The requirement to provide samples of substantially pure DNA free from cellular proteins may arise in a number of instances where there is a need to investigate the detailed DNA structure of an individual.

In the field of forensic science, the technique of genetic fingerprinting involves the identification of individual persons on the basis of their unique DNA structure. Within a person's DNA are a number of "hypervariable regions" which are unique to the individual, yet include common core sequences. No two individuals share the same set of hypervariable regions (except identical twins). The fingerprint is produced by controlled digestion of the DNA using a set of restriction enzymes, slab gel electrophoresis and Southern blotting, followed by extraction of restriction fragment length polymorphisms using hybridisation probes specific to the core sequences.

In order to carry out genetic fingerprinting, a sample of substantially pure DNA is required and this is usually obtained from a sample of whole blood. The blood sample is processed by separation of the lymphocytes therefrom, lysis of the lymphocytes to yield mixtures of DNA and proteins, and separation of the DNA from the mixture. Since the available blood sample (for example, from the scene of a crime) may be only small, it is important that the DNA isolation procedure be as efficient as possible.

Techniques are becoming available for the diagnosis of genetically related diseases which depend on investigating the DNA structure for genetic defects. Tests may be carried out on putative parents where one or both parent has a family history including the disease, in order to establish the likelihood of the baby having the disease.

Cellular material may also be obtained from fetal blood samples or samples of amniotic fluid for direct diagnosis of genetic disorders prior to birth. In the future there is the hope that gene therapies may become available for remedying the missing or mutant gene.

In conventional DNA isolation processes, the lymphocytes are lysed using a lysis buffer containing sodium dodecyl sulphate (SDS) to solublise the cell membranes. The lysis mixture contains DNA and cellular proteins, possibly in intimate association. The mixture is incubated overnight at 370C with proteinase K which digests the proteins. The SDS buffer also acts to activate proteinase K. The proteins are digested into smaller peptide fragments by fission at the peptide bonds. The protein fragments are then denatured and removed from the mixture employing sequential extractions using phenol, phenol/chloroform mixture and chloroform. The function of the phenol is to denature the proteins. Use of phenol/chloroform mixtures inhibits RNase enzyme activity. The final extraction with chloroform (using chloroform/isoamyl alcohol 24:1 v/v) removes any residual traces of phenol from the DNA preparation. Finally, the DNA is concentrated using absolute alcohol.

There are a number of problems associated with the conventional method. Firstly, the overnight incubation with proteinase K causes partial degradation of high molecular weight DNA. This is due to the lengthy incubation period during which the DNA is exposed to degradation resulting from any residual cellular nucleases present in the lysis mixture or from the proteinase K preparation used. Also phenol is toxic and its use poses a safety hazard to operators, and there are associated problems of safe disposal.

Furthermore, the yield of DNA is relatively low and can only be improved by time-consuming repeated extractions.

Extraction with chloroform results in a lower protein-containing chloroform layer and an upper aqueous layer containing DNA. However, the separation into layers is incomplete and an intermediate aqueous layer containing DNA and protein tends to be present, which makes the efficient separation of the aqueous layer from the organic layer very difficult. This can only be achieved by repeated solvent extractions. Moreover, the DNA which is always present in the upper layer, is usually withdrawn by suction and the shear forces associated with this technique tend to cause degradation of the DNA especially when repeated extractions are required. Even with the repeated time consuming extractions, the maximum purity obtainable is reflected in an absorbance ratio of about 1.7. This is the ratio of absorbance at 260 nm (DNA) to that at 280 nm (protein).

US-A-4923978 discloses processes for the separation of protinaceous material from nucleic acids utilising a solid phase support to immobilise and hence remove proteins. The solid phase support is for example a derivatised silica gel which has been treated with an oxysilane. The process is simple and avoids the use of organic solvents thereby eliminating a source of contamination in previous separation processes for nucleic acids.

It is an object of the present invention to provide an improved process involving a two-phase extraction system utilising an organic solvent for the isolation of DNA by removal of proteins from DNA/protein mixtures.

According to the present invention there is provided a process for the isolation of polynucleotides from an aqueous mixture of the polynucleotides and proteins contained in a holding tube, said process comprising
firstly forming a two phase solvent extraction system by adding to the tube and mixing with the aqueous mixture therein an immiscible organic solvent which extracts the proteins and results in a lower protein-containing solvent layer, an upper aqueous layer containing polynucleotides and an intermediate aqueous layer containing polynucleotides and proteins, and
secondly scavenging proteins from the aqueous layers by adding to the two phase system in the tube, without mixing of the layers, a powder of polymerised silica gel particles containing free aldehyde (-CHO) or ketone (=CO) groups,
whereby the particles absorb proteins from the intermediate aqueous layer and form a solid disc adherent to the walls of the tube at the interface between the aqueous and organic solvent layers so that thereafter the aqueous polynucleotide containing layer can be completely removed from the tube without contamination from the protein-containing solvent layer.

The protein absorbing material may be prepared by cross-linking a hydroxyl-containing substrate material. Such substrate materials are well known in the field of chromatography and the present invention employs a silica.

Cross-linking may be carried out using a cross-linking agent which either contains CHO or CO groups, or which can be reacted further so as to introduce free CHO or CO groups. Preferably, the cross-linking agent comprises polyalkoxy groups which react with the free hydroxyl groups on the substrate, eliminating alcohol. The present invention preferably employs an alkoxysilane of general formula (I)

(R¹0)₃ Si - R² - NH₂ (I)

Where
R¹ is C₁-C₆ alkyl, and
R² is C₁-C₆ alkylidene.

The preferred cross-linking agent is 3-aminopropyltriethoxysilane. This compound contains an amino substituent which may be further reacted to introduce free CHO or CO groups, for example, by reaction with a dialdehyde or haloaldehyde of general formula (II).

X- (CH₂)ₙ - CR³0 (II)

where
X is CR³ 0 or halo (preferably chloro).
R³ is H or C₁-C₆ alkyl, and
n is 3 to 10 (preferably 3 to 5).

The preferred reagent is glutaraldehyde OHC (CH₂)₃ CHO. The value of n may be varied to vary the chain length in order to improve access of the proteins to the CHO or CO groups respectively.

The resulting cross-linked protein absorbing material is solid or semi-solid to facilitate use in separation from the DNA-containing solution. Additionally, reaction of the free CHO or CO groups with digested or undigested protein also results in some cross-linking.

The protein cross-linking step produces a solid mass of cross-linked material, thereby facilitating separation.

The solid mass of material containing absorbed protein has a density different from the DNA-containing solution and isolation of DNA is achieved in the two-phase solvent extraction system, wherein the aqueous DNA solution is present as the upper layer. The density of the material containing absorbed protein is arranged such that it forms an intermediate layer between the upper aqueous layer and the lower solvent layer, so as to assist complete removal of the aqueous DNA-containing layer without contamination from the solvent layer.

A silica-based protein absorbing material is used, and it is preferred to use silica gel of a particle size 250-400 mesh in order to provide a solid cross-linked mass when reacted with protein.

The DNA is generally derived from lymphocytes, though the invention may also be applied to DNA obtained from other cell types. The technique of the present invention is also applicable to the separation of oligonucleotides in general from proteins. For example, the technique may be used to separate DNA restriction fragments from mixtures with restriction enzymes (which are proteins). Such mixtures are produced for example in the digestion of DNA by restriction enzymes in order to produce restriction fragments for gel electrophoresis separation in DNA fingerprinting procedures.

The lysis of the cells is preferably carried out using a surfactant, such as SDS buffer.

Since at least some of the DNA and protein present in the lysis mixture may be intimately associated, it is preferred to denature the proteins (e.g. by heating). Although not essential, it is also preferred to digest the proteins to reduce their chain length prior to treatment with the protein absorbing material. This may be carried out using proteinase K and chloroform as in the conventional method; but this suffers from the disadvantage described earlier in this specification. In a preferred embodiment of the invention, a mild oxidising agent such as sodium perchlorate (optionally together with a surfactant and heating but freshly prepared and filtered (0.25-0.45 microns)) is used to denature and digest the proteins. Contrary to previous belief, it has been surprisingly found that mild oxidising agents may be employed without degrading the DNA itself.

The preferred embodiment allows the isolation of DNA to be carried out much more speedily than using the conventional method. Moreover, the DNA is obtained in high yield and with improved purity.

Embodiments of the present invention will now be described by way of example only.

### Example 1 - PREPARATION OF PROTEIN-ABSORBING MATERIAL (Polymerisation of Silica Gel)

50 grams of dried particulate silica gel (250-400 mesh) was activated by addition of 300 mL of 5% nitric acid. One hour later the silica gel was washed with 1000 mL of double distilled water and then 400 mL of HPLC grade methanol using a vacuum pump.

The pH value of 300 mL of 10% 3-aminopropyltriethoxysilane (C₂H₅O)₃-S-CH₂CH₂CH₂ NH₂ (APTES) was adjusted to 3.5 with 6N hydrochloric acid. The solution was then added to the above silica gel. The reactants were put in a 750C water bath for 2 hours and then washed with 500 mL of double distilled water. The treated silica gel was then dried in a 115°C oven for at least 5 hours.

The pH value of 400 mL aqueous glutaraldehyde OHC-(CH₂)₃-CHO (2.5%) was adjusted to 7.0 and the solution was then added to the treated silica gel. The reaction was allowed to proceed at room temperature for 2 hours with stirring. The resulting polymerised silica gel was then thoroughly washed with 1000 mL of deionised double distilled water, and then drained using a vacuum pump, to yield a free-flowing powder.

### Example 2 - ISOLATION OF DNA FROM LYMPHOCYTES

The removal of proteins (mainly histone proteins associated with DNA) present in the lysis solution is a key step in the purification of genomic DNA and was carried out as follows.

### (a) Preparation of Lymphocytes

To a 2.0-mL volume autoclaved Eppendorf tube was added 550µL EDTA treated whole blood (500µL whole blood + 50µL of 2% aqueous EDTA) and 1.45 mL of buffer A (lymphocytes extraction buffer). The mixture was left at room temperature for 6.0 min with occasional gentle inversion of the containing vessel and then centrifuged at 2040 x g for 4.0 min. The supernatant was removed immediately to leave a lymphocyte cell pellet. Experiment shows that it is best to use small diameter tubes, e.g. 2mL tube 7-8mm diameter.

| | | | |
|---|---|---|---|
| Buffer A : | 10 mM | Tris-HCl | |
| | 320 mM | Sucrose | |
| | 5 mM | MgCl₂ | |
| | 1% | Triton X-100, | pH 7.5 |

### (b) Lysis of Lymphocytes

The isolated lymphocyte cell pellet was resuspended in 500µL of buffer B (lysis buffer) and 125 µL of 5 M sodium perchlorate solution was added at once. After being well mixed gently, the content was heated at 370C for 20 mins and then at 60°C for a further 20 mins before being cooled. This treatment caused lysis of the lymphocytes, denaturing and partial digestion of the protein to provide a lysis mixture.

| | | | |
|---|---|---|---|
| Buffer B : | 400 mM | Tris-HCl | pH 8.0 |
| | 60 mM | EDTA | |
| | 150 mM | NaCl | |
| | 1% | SDS | |

### (c) Isolation and Concentration of DNA

An equal volume of chloroform was added to the lysis mixture obtained above and subjected to very gentle inversion of the containing vessel for 5 mins and then spun at 82 x g for 2 mins (horizontal centrifuge). After adding 120 mg of protein absorbing material in the form of polymerised silica gel powder produced in Example 1 without any mixing, the mixture was centrifuged at once at 735 x g for 2 mins. If possible it is preferred to use horizontal centrifugation. The amount of polymerised silica gel which is used is partly dependent upon the diameter of the analytical tubes used for extraction. The amount cited refers to tubes of 2 mL volume having a diameter of 7-8mm. It is sufficient if a 1-2mm layer thickness is formed after centrifugation for success of the technique. After the tube was removed from the centrifuge it was seen that the silica gel was positioned between the top aqueous layer (containing the desired DNA) and the bottom organic layer as a hard disk adhered to the tube wall with proteins trapped inside. The aqueous layer was transferred to another 2-mL volume tube and two volumes of 40C absolute alcohol was immediately added. After the mixture was gently mixed for 15 secs. by inverting the tube, a fibrous network form of DNA was precipitated out and the supernatant was removed. Then 95% alcohol was used to wash the DNA pellet. The supernatant alcohol was removed and the purified DNA was dried in a vacuum dessicator for 10 min. The purified and dried DNA was finally redissolved in 50µL of TE buffer and was ready for DNA fingerprinting.

| | | | |
|---|---|---|---|
| TE Buffer : | 10 mM | Tris-HCl | |
| | 1 mM | EDTA | pH 7.5 |

### (d) RESULTS

The whole process took less than two hours instead of 8 to 15 hours using the conventional method. The yield of DNA was about 10 µg per 500 µl of whole blood which demonstrates approximately a 100% improvement over the more usual 4.0 to 6.0 µg per 500 µl of whole blood obtained from the conventional extraction procedure. Moreover, the high purity of the DNA produced by this new one step method is attested to by the consistently obtained value of 1.8 or higher for the UV absorbance ratio of DNA at 260 mm to protein at 280 mm compared with normal values of 1.3 to 1.4 obtained by the conventional method. Since these values are expressed on a logarithmic scale, the purities obtained according to the present invention are in fact approximately twice as high as those normally obtainable using conventional techniques. Values of 1.7 are the best which can be obtained by the conventional method but this requires several deleterous transfers. In addition the new method permits the processing of 45 µl samples of whole blood in contrast to the 60 µl of the conventional method.

By the running of mini-gel electrophoresis it can be seen that the extracted DNA shows fewer degradations than when the conventional method is used.

## Claims

1. A process for the isolation of polynucleotides from an aqueous mixture of the polynucleotides and proteins contained in a holding tube, said process comprising
firstly forming a two phase solvent extraction system by adding to the tube and mixing with the aqueous mixture therein an immiscible organic solvent which extracts the proteins and results in a lower protein-containing solvent layer, an upper aqueous layer containing polynucleotides and an intermediate aqueous layer containing polynucleotides and proteins, and
secondly scavenging proteins from the aqueous layers by adding to the two phase system in the tube, without mixing of the layers, a powder of polymerised silica gel particles containing free aldehyde (-CHO) or ketone (=CO) groups,
whereby the particles absorb proteins from the intermediate aqueous layer and form a solid disc adherent to the walls of the tube at the interface between the aqueous and organic solvent layers so that thereafter the aqueous polynucleotide containing layer can be completely removed from the tube without contamination from the protein-containing solvent layer.

2. The process of Claim 1, wherein immediately after the powder is added the tube is centrifuged.

3. The process of either preceding Claim, wherein the protein-reactive silica gel particles have a mesh size in the range 250 - 400.

4. The process of any preceding Claim, wherein the material which is reactive to proteins has been prepared by
(i) cross-linking silica gel particles with an alkoxysilane of general formula (I)
(R¹O)₃ Si-R²-NH₂ (I)
where R¹ is C₁₋₆ alkyl, and R² is C₁₋₆ alkylidene;
and (ii) reacting the cross-linked particles with an amino-reactive compound of general formula (II)
X-(CH₂)ₙ -CR³O (II)
where X is CR³O or halo; R³ is H or C₁₋₆ alkyl; and n is 3 to 10.

5. The process of Claim 4, wherein the cross-linking agent of general formula (I) is 3-aminopropyltriethoxysilane; and the amino-reactive compound of general formula (II) is glutaraldehyde.

6. The process of Claim 5 including the further steps of removing the polynucleotide-containing aqueous layer from the tube and precipitating out the polynucleotides, and wherein the UV absorbance ratio in the precipitate of polynucleotides at 260 nm to protein at 280 nm is at least 1.8.

## Patentansprüche

1. Verfahren zum Isolieren von Polynucleotiden aus einem wäßrigen Gemisch aus Polynucleotiden und Proteinen, das in einem Vorratsröhrchen enthalten ist, wobei das Verfahren folgende Schritte umfaßt:
Erstens wird durch Eingeben eines nicht mischbaren organischen Lösungsmittels in das Röhrchen und Mischen mit dem wäßrigen Gemisch darin ein Zweiphasen-Lösungsmittelextraktionssystem gebildet, wobei das Lösungsmittel die Proteine extrahiert sowie eine untere proteinhaltige Lösungsmittelschicht, eine obere wäßrige Schicht mit einem Gehalt an Polynucleotiden und eine dazwischenliegende wäßrige Schicht mit einem Gehalt an Polynucleotiden und Proteinen ergibt, und
zweitens werden die Proteine durch Versetzen des Zweiphasensystems in dem Röhrchen, ohne die Schichten zu vermischen, mit einem Pulver aus Teilchen eines polymerisierten Kieselgels, das freie Aldehydgruppen (-CHO) oder Ketogruppen (=CO) enthält, aus den wäßrigen Schichten herausgespült,
wobei die Teilchen Proteine aus der dazwischenliegenden wäßrigen Schicht absorbieren und eine feste Scheibe bilden, die an der Grenzfläche zwischen der wäßrigen Schicht und der Schicht des organischen Lösungsmittels derart an den Röhrchenwänden haftet, daß anschließend die polynucleotidhaltige wäßrige Schicht ohne Verunreinigung durch die proteinhaltige Lösungsmittelschicht vollständig aus dem Röhrchen entnommen werden kann.

2. Verfahren nach Anspruch 1, wobei unmittelbar nach der Zugabe des Pulvers das Röhrchen zentrifugiert wird.

3. Verfahren nach irgendeinem der vorstehenden Ansprüche, worin die Teilchen des proteinreaktiven Kieselgels eine Maschengröße im Bereich von 250 bis 400 aufweisen.

4. Verfahren nach einem der vorstehenden Ansprüche, worin das Material, welches gegenüber Proteinen reaktionsfähig ist, durch folgende Schritte hergestellt worden ist:
i) Vernetzen von Kieselgelteilchen mit einem Alkoxysilan der allgemeinen Formel I
(R¹O)₃ Si-R²-NH₂ (I),
worin R¹ ein C₁₋₆-Alkyl und R² ein C₁₋₆-Alkyliden bedeutet, und
ii) Umsetzen der vernetzten Teilchen mit einer aminoreaktiven Verbindung der allgemeinen Formel II
X-(CH₂)ₙ-CR³O (II),
worin X den Rest CR³O oder ein Halogen, R³ den Rest C₁₋₆-Alkyl oder H und n eine Zahl von 3 bis 10 bedeuten.

5. Verfahren nach Anspruch 4, worin das Vernetzungsmittel der allgemeinen Formel I 3-Aminopropyltriethoxysilan und die aminoreaktive Verbindung der allgemeinen Formel II Glutaraldehyd ist.

6. Verfahren nach Anspruch 5, einschließlich der weiteren Stufen des Entfernens der polynucleotidhaltigen wäßrigen Schicht aus dem Röhrchen und des Ausfällens der Polynucleotide, wobei das UV-Absorptionsverhältnis in der Fällung der Polynucleotide bei 260 nm zum Protein bei 280 nm mindestens 1,8 beträgt.

## Revendications

1. Procédé d'isolement de polynucléotides à partir d'un mélange aqueux de polynucléotides et de protéines contenus dans un tube de rétention, ledit procédé comprenant :
- premièrement, la formation d'un système d'extraction par solvant à deux phases, par addition d'un solvant organique non miscible qui extrait les protéines, et qui donne une couche inférieure contenant les protéines, une couche aqueuse supérieure contenant les polynucléotides, et une couche aqueuse intermédiaire contenant des polynucléotides et des protéines, et
- deuxièmement, l'épuration des protéines à partir des couches aqueuses, en additionnant au système à deux phases dans le tube, sans mélanger les couches, une poudre de particules de gel de silice polymérisée contenant des groupes aldéhyde (CHO) ou cétone (=CO) libres,
grâce à quoi les particules absorbent les protéines provenant de la couche aqueuse intermédiaire, et forment un disque solide adhérant aux parois du tube, à l'interface entre les couches aqueuse et solvant organique, de sorte que par la suite, la couche aqueuse contenant les polynucléotides peut être complètement sortie du tube sans contamination de la part de la couche solvant contenant les protéines.

2. Procédé selon la revendication 1, dans lequel immédiatement après que la poudre ait été ajoutée, le tube est centrifugé.

3. Procédé selon l'une quelconque des revendications précédentes, dans lequel les particules de gel de silice réactives vis-à-vis des protéines, ont une taille correspondant à l'intervalle 250-400 mesh.

4. Procédé selon l'une quelconque des revendications précédentes, dans lequel le matériau qui est réactif vis-à-vis des protéines a été préparé par :
(i) réticulation de particules de gel de silice avec un alcoxysiloxane de formule générale (I) :
(R¹O)₃Si-R²-NH₂ (I)
dans laquelle R¹ est un groupe alkyle C₁₋₆, et R² est un groupe alkylidène C₁₋₆ ; et
(ii) réaction des particules réticulées, avec un composé amino-réactif de formule générale (II) :
X-(CH₂)ₙ-CR³O (II)
dans laquelle X représente CR³O ou un halogène ; R³ représente un atome d'hydrogène ou un groupe alkyle C₁₋₆ ; et n varie de 3 à 10.

5. Procédé selon la revendication 4, dans lequel l'agent de réticulation de formule générale (I) est le 3-aminopropyltriéthoxysilane; et le composé amino-réactif de formule générale (II) est le glutéraldéhyde.

6. Procédé selon la revendication 5, comprenant l'étape additionnelle de séparation depuis le tube, de la couche aqueuse contenant les polynucléotides, et de précipitation des polynucléotides, et dans lequel le ratio entre l'absorbance UV à 260 nm, dans le précipité de polynucléotides, et l'absorbance UV à 280 nm, des protéines, est d'au moins 1,8.
